# EUROPEAN PATENT APPLICATION

(11) **EP 1 352 609 A1**
(43) Date of publication of application: **15.10.2003**
(21) Application number: 03007476.9
(22) Date of filing: 07.04.2003
(51) Int. Cl.: A61B 5/00

(54) **Method and apparatus for remote transmission of data, information and instructions between patients and specialized personnel**

(30) Priority: 11.04.2002 IT BO20020191
(71) Applicant: Sined S.r.l., 40057 Granarolo dell'Emilia, (Prov. of Bologna) (IT)
(72) Inventor: Rustici, Andrea, 20020 Lainate (Prov. of Milano) (IT); Calletti, Filippo, 40125 Bologna (IT); Betuzzi, Giovanni, 42023 Ca del Bosco di Sopra (IT); Rossi, Marco, 41030 Medolla (Prov. of Modena) (IT)
(74) Representative: Modiano, Guido, Dr.-Ing.

(57) **Abstract**

A method for the remote transmission of a plurality of items of information and instructions between remote patients and specialized personnel, comprising the steps of : providing the patient with an identification code (*an*); establishing a number of questions (d1, d2, ... dk) correlated to the clinical situation of the patient, to which the patient must give successive answers (ra, r1, r2, r3, ... rk), which constitute a packet (Pn) of specific data of a certain patient at a certain moment; encoding said packet (Pn) in the SMS format by keying in the data on a mobile terminal (TM); and sending it over the mobile telephone network by way of an integrated GSM aerial to a central unit (U) which is provided with an electronic computer (E) adapted to acquire said packet (Pn).

## Description

The present invention relates to a method for remote transmission of a plurality of data, information and instructions between remote patients and specialized personnel, and to a portable and fixed apparatus for performing the method.

For the management of long-term and terminal patients, such as for example cardiopathics, diabetics or patients undergoing dialysis, it is economically and socially more advantageous to perform the treatment at home rather than proceed with hospitalizations.

In many cases, certain functional parameters of the patient must be monitored, one must ascertain that certain prescriptions have been followed, and it must be possible to evaluate the effectiveness of certain prescriptions as a function of the reactions of the patient's body.

In relation to this, there is the widespread practice of entrusting the patient at home with devices such as blood pressure measurement devices, a thermometer, scales, glycemia measurement units, or dispensers of substances such as oxygen, drip feeds, injections, tablets, et cetera.

The effectiveness of medical treatments depends to a large extent on the regular monitoring of the life parameters of the patient and on the regular taking of the prescribed foods or medicines: however, it is difficult to send to the patient's home specialized personnel in order to monitor, perform and evaluate the result of the treatments, also because this would entail the need for the patient to be often, and at certain times, in places that can be reached easily by medical or paramedic personnel.

The need is particularly felt to be able to keep the patients under medical observation while leaving them the greatest freedom of movement and discretion in the choice of intervention times

The aim of the present invention is to meet these needs, by providing a method for the remote transmission of a plurality of data, information and instructions between home patients and specialized personnel and a portable and fixed apparatus for performing the method, which allows maximum freedom of movement to the patient and maximum freedom in choosing the moment when the physician performs his study and intervention.

Within this aim, an object of the present invention is to provide a method and an apparatus that is simple, relatively easy to provide in practice, safe in use, effective in operation, and relatively low in cost.

This aim and this object are achieved by the present method for the remote transmission of a plurality of items of information and instructions between remote patients and specialized personnel, characterized in that it comprises the steps of: providing the patient with an identification code; establishing a number of questions correlated to the clinical situation of the patient, to which the patient must give successive answers, which constitute a packet of specific data of a certain patient at a certain moment; encoding said packet in the SMS format by keying in the data on a mobile terminal; and sending it over the mobile telephone network by means of an integrated GSM aerial to a central unit which is provided with an electronic computer adapted to acquire said packet.

Further features and advantages will become better apparent from the detailed description of a preferred but not exclusive embodiment of a method for the remote transmission of a plurality of data, information and instructions between remote patients and specialized personnel, and of a portable and fixed apparatus for performing the method according to the invention, illustrated only by way of non-limitative example in the accompanying drawings, wherein:

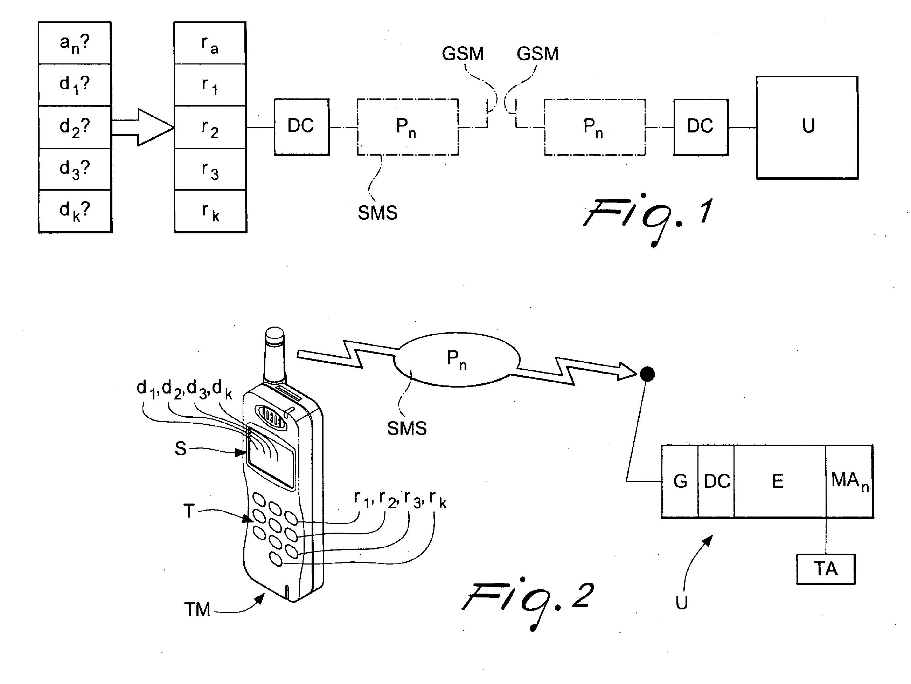

Figure 1 is a block diagram of the method for the remote transmission of a plurality of data, information and instructions between remote patients and specialized personnel;
Figure 2 is a schematic view of the apparatus for performing the method according to the invention.

With reference to the figures, lowercase letters designate hereinafter the steps of the method for the remote transmission of a plurality of data, information and instructions between remote patients and specialized personnel, and uppercase letters designate the components of the portable and fixed apparatus for performing the method.

The method consists in providing each patient *n* with an identification code *an*, in establishing a succession of questions d1, d2, d3, ... dk, which advantageously appear one after the other on the screen S of a mobile terminal TM and are correlated to the clinical situation of the patient, and to which the patient must give successive answers.

Here is an example of a table of questions and/or preset clinical parameters:
-- Body weight
-- Systolic pressure
-- Diastolic pressure
-- Heart rate
-- Total infused volume
-- Type of solution
-- Number of daily exchanges
-- Rate of infusion
-- Duration of infusion
-- Volume of drained liquids
-- Duration of drainage
-- Retention time
-- Type of set used
-- Body temperature
-- Glycemia
-- Patient's symptoms
-- Complications of the treatment
-- Detected machine alarms
-- Results of Cytur test
-- Drugs taken and doses thereof
-- Prescription of new drugs
-- Request for telephone contact
-- Confirmation that treatment is adequate

The successive answers ra, r1, r2, r3, ... rk form a packet Pn of clinical data that are specific to a certain patient at a certain time; the packet Pn is encoded in SMS format by entering the data on the keypad T of a mobile terminal TM and is sent over the mobile telephone network by means of an integrated GSM aerial to a central unit U provided with an electronic computer E adapted to acquire the packet Pn.

In the electronic computer, the data packet is allocated in a memory portion MAn that is assigned to a certain patient, from which it can be retrieved, processed and studied by specialized personnel authorized to evaluate the clinical data and, optionally, transmit over the mobile telephone network to the telephone set of the patient instructions regarding food or drugs to be taken or discontinued, and regarding the subsequent data packet to be sent.

The apparatus for performing the method is constituted by a mobile telephone set TM with a dedicated screen that is compatible with the SMS transmission standard, on which the original resident program is replaced by a management program that runs constantly and is adapted to propose, by means of a series of basic graphic images displayed on the screen, successive questions d1, d2, d3, ... dk in relation to the pathological or clinical situation or to values read on any devices and in relation to the taking of food or medical substances, to which the patient must progressively enter the answers r1, r2, r3, ... rk and which, when the answers are completed, sends the acquired data packet to the central unit.

The central unit is constituted by an electronic computer E, which comprises a unit G for connection to the mobile telephone network provided with an integrated GSM aerial, a DC decoding and encoding unit designed to convert the SMS format in which the data of the packet are received and transmitted into a format that is adapted to be displayed and analyzed and vice versa, a keyboard T on which the instructions to be transmitted to the patient can be keyed.

The devices supplied to the customer can be of the automatic, semiautomatic and manual type.

Operation of the invention is intuitive, in that the data related to a certain patient are transmitted in the SMS format, which ensures the anonymity of the transmission and the confidentiality of the doctor-patient relationship and allows freedom of movement to the patient and discretion regarding the moment when the physician intervenes.

It has thus been shown that the invention achieves the intended aim and object.

The invention thus conceived is susceptible of numerous modifications and variations, all of which are within the scope of the appended claims.

All the details may further be replaced with other technically equivalent ones.

In practice, the materials used, as well as the shapes and the dimensions, may be any according to requirements without thereby abandoning the scope of protection of the appended claims.

The disclosures in Italian Patent Application No. BO2002A000191 from which this application claims priority are incorporated herein by reference.

## Claims

1. A method for the remote transmission of a plurality of items of information and instructions between remote patients and specialized personnel, **characterized in that** it comprises the steps of : providing the patient with an identification code (*an*); establishing a number of questions (d1, d2, ... dk) correlated to the clinical situation of the patient, to which the patient must give successive answers (ra, r1, r2, r3, ... rk), which constitute a packet (Pn) of specific data of a certain patient at a certain moment; encoding said packet (Pn) in the SMS format by keying in the data on a mobile terminal (TM); and sending it over the mobile telephone network by way of an integrated GSM aerial to a central unit (U) which is provided with an electronic computer (E) adapted to acquire said packet (Pn).

2. The method according to claim 1, **characterized in that** said packet is acquired by said computer (E), with preliminary analysis and checking of the consistency of the data contained in the packet (Pn).

3. The method according to claim 1, **characterized in that** in said electronic computer (E) said packet (Pn) is allocated in a portion of memory (MAn)assigned to the patient, from which it can be retrieved, processed and studied by specialized personnel authorized to evaluate the data and optionally transmit over the mobile telephone network to the mobile terminal (TM) of the patient instructions regarding the foods or drugs to be taken or discontinued and regarding the subsequent data packet (Pn) to be sent.

4. An apparatus for performing the method, **characterized in that** said mobile terminal is constituted by a mobile telephone set (TM) with a dedicated screen that is compatible with the SMS transmission standard, on which the original resident program is replaced with a management program that is run permanently and is adapted to propose, by way of a series of basic graphic images displayed on the screen (S), successive questions in relation to the pathological or clinical condition or to values read on any devices and in relation to the taking of food or medicines to which the patient must progressively key in the answers and which, when the answers are completed, sends the packet (Pn) of acquired data to the central unit (U).

5. The apparatus according to claim 4, **characterized in that** said central unit (U) is constituted by an electronic computer (E), which comprises a unit (G) for connection to the mobile telephone network which is provided with an integrated GSM aerial, a decoding and encoding unit (DC) adapted to convert the SMS format in which the data of said packet (Pn) are received and transmitted into a format that is adapted to be displayed and analyzed and vice versa, and a keyboard (T) on which it is possible to type the instructions to be transmitted to the patient.
